(19) Europäisches Patentamt

European Patent Office

Office européen des brevets

(11) Publication number: **0 245 338 B1**

(12) **EUROPEAN PATENT SPECIFICATION**

(45) Date of publication of patent specification: **22.01.92**

(51) Int. Cl.⁵: **C12N 11/02**, C12N 11/14, C12N 5/00

(21) Application number: **86906386.7**

(22) Date of filing: **22.10.86**

(86) International application number: **PCT/GB86/00653**

(87) International publication number: **WO 87/02703 (07.05.87 87/10)**

(54) MICROCARRIER FOR CELL CULTURE.

(30) Priority: **22.10.85 GB 8526096**

(43) Date of publication of application: **19.11.87 Bulletin 87/47**

(45) Publication of the grant of the patent: **22.01.92 Bulletin 92/04**

(84) Designated Contracting States: **BE CH DE FR GB IT LI NL SE**

(56) References cited:

**See also references of WO8702703**

(73) Proprietor: **NATIONAL RESEARCH DEVELOP-MENT CORPORATION**
**101 Newington Causeway**
**London SE1 6BU(GB)**

(72) Inventor: **ROBINSON, Eric**
**146 Moss Road**
**Lambeg County Antrim BT27 4LF(GB)**

(74) Representative: **Neville, Peter Warwick**
**Patent Department National Research Devel-opment Corporation 101 Newington Cause-way**
**London SE1 6BU(GB)**

Rank Xerox (UK) Business Services

## Description

This invention relates to microcarriers for cell culture. In this specification, the words "microcarrier" and "carrier" are used interchangeably.

The culture of anchorage-dependent animal or plant cells is enhanced if a large and appropriate surface area for anchorage is provided within the culture environment. Various microcarriers have been developed for this purpose, for example 200-micron beads of cross-linked dextran with a derivatised surface. These are suspended in the culture medium and provide an attractive surface for the attachment and proliferation of animal cells. Using such carriers, higher yields of cells can be obtained per unit volume of the culture and improvements made in the production of cell products and biologically active substances such as vaccines or interferon. Other uses of such carriers are as chromatography column-packing beads or in other separation processes.

According to the present invention, a carrier for living cells comprises an indented body, being an aggregation of microparticles, preferably microbeads such as conveniently of aluminosilicate or silica, from 2-30 e.g. 15-15 microns across, the aggregation having interstices between the microparticles, the body bearing an organic polymer coating providing an attractive surface for cell adhesion. The body may be generally rounded and from 50 to 1000, preferably 100 to 400 more preferably 125-250 microns across, or from 30 to 110, preferably 40 to 80, microparticles across, and may have a creviced surface, the crevices themselves also being indented.

The microparticles may be fused together at their points of contact, or held together merely by air-drying at 50C-100C e.g. 60C, with the organic polymer being applied subsequently, or the microparticles may first be coated with the polymer, the polymer then holding the aggregation together. This latter method is especially suitable for the creviced structure, which may be controlled according to the looseness or otherwise of the packing of the microparticles. The indentations (in the case of an aggregation of microparticles, the interstices) will preferably be roughly in the region of 1-20 such as 2-10 microns across, and in the case of interstices account preferably for 30-80% of the volume of the carrier. The crevices if present might be from 3-10 indentations deep and from 2 to 5 indentations wide.

The organic polymer preferably has a surface charge of at least $10^{15}$ charges/cm$^2$, and/or an exchange capacity of at least 0.2 preferably at least 0.5 microequivalents per gram. The polymer may be in a layer, porous or non-porous, from 1 to 30 molecules thick, and preferably contains amino or hydroxyl groups, which should be insoluble in normal nutrient media.

The indentations provide sheltered recesses in which individual cells can anchor themselves and grow, despite a maybe turbulent environment for suspending the carriers in a liqiud nutrient medium. The indentations also help to protect the resident cells from crushing or dislodgment by impact with other carriers.

Where made from microparticles, the carrier according to the invention may not only provide an attractive surface for cell growth but also a high internal porosity which can act as a reservoir or supply-route for nutrients. Cells growing on the carrier surface would thus be provided with essential nutrients from all sides.

This invention provides a method of culturing cells (such as plant, animal or fungal), comprising providing the cells with nutrient and carriers according to the invention as set forth above wherein the indentations or the microspheres are preferably from half to twice the diameter of the cells. The cells may be harvested by degrading the said polymer. In this way, the cells simply drop off the carrier and can be collected, which is far less destructive than the previously known method of enzymatically degrading the cell wall to release the cell from the substrate to which it had been anchored.

A suitable silica for microbeads may be prepared according to USA Patent 4752458 and PCT Publication WO 86/07345 wherein the internal pore structure and density may be controlled. The carriers preferably have a pore volume of 0.3 to 0.8 ml/ml. The silica may be treated with an aluminium salt solution to form a surface aluminosilicate, or it may be methylated to remove surface hydroxyl groups. The carrier, though preferably somewhat acidic, could however be of almost any sufficiently rigid material such as a metal oxide or a carbide or gelatin, especially if the polymer coating is non-porous and thus 'conceals' the carrier material from the cells.

The overall specific density of the carriers in use (= density in g/cm$^3$) is preferably from 1.1 to 1.6, such as 1.2 to 1.5. The higher densities are surprisingly beneficial, as the carriers settle out of the nutrient medium more easily. Although in use this means that they require more vigorous stirring to keep them in suspension than do prior-art carriers of specific density 1.05 or so, the indentations protect the cells from shear and turbulence.

The carrier is preferably rounded e.g. spheroidal, preferably overall generally spherical or oval in shape.

Such carriers may be formed from larger irregularly shaped particles which are aggregations of microparticles by vibrating a mass of such particles together in a suitable container where by attrition the edges and corners are ground away, and the carrier size may be controlled by extruding such a mass of aggregations (e.g. filter-cake) through a sieve of appropriate mesh. Further size discrimination to have all carriers within 25% of the desired size is possible by sedimentation.

Suitable organic polymers for providing a porous or non-porous coating (before or after the microparticles are aggregated if that route is followed) to form the cell-attracting and retaining surface include agar, alginic acid, cellulose or its acetate or nitrate, chitosan, collagen, dextran, gelatin, glycogen, kappa-carrageenan polystyrene, polyvinyl acetate, agarose, casein, gum tragacanth, guar gum, gum xanthan and pectin or derivatives of these. If appropriate these may be cross-linked with an agent such as an aldehyde such as formaldehyde or glutaraldehyde to strengthen or insolubilise the coating.

A particularly suitable culture surface is provided by chitosan, a partially deacetylated chitin which contains free amino groups. This may be applied by adding silica particles to a solution of chitosan in 1-5% formic acid such that all the solution is taken up on the surface of the carrier, preferably porous silica. The mass is then added to a vigorously stirred solution of dilute sodium or ammonium hydroxide to precipitate a surface coating of chitosan on the silica particles.

Where a thinner layer is required this may be achieved by suspending the silica particles in water and, while stirring, adding a solution of chitosan in formic or acetic acid. The surface layer of chitosan forming on the silica particles is insolubilised by changing the pH, conveniently by adding dilute ammonium or sodium hydroxide.

Similar techniques may be used to apply other polymers. Thus cellulose is applied as a surface coating using a solution of cellulose in cuprammonium hydroxide while alginic acid is applied as sodium alginate which is insolubilised by adding to a dilute acid solution.

The polymer may be pervious or impervious or semi-permeable, for example allowing N, P and K (nutrients) to pass while barring proteins.

Surface layers of agar, gelatin and kappa-carrageenan are applied by thermal gelation followed by cross-linking.

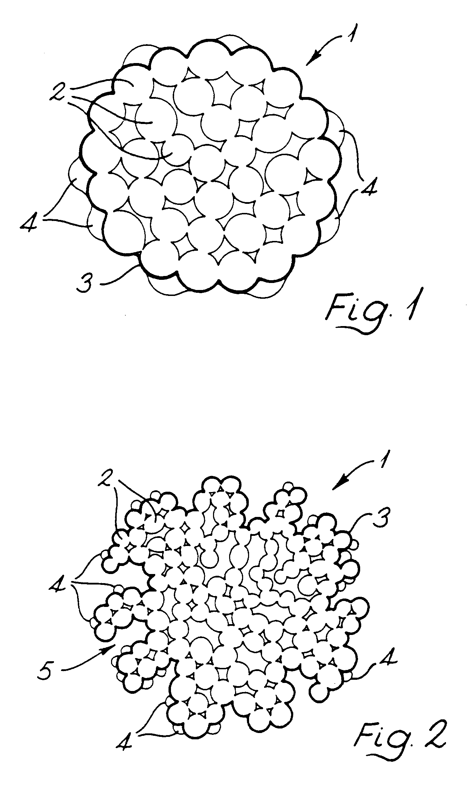

A specific embodiment of the invention will now be described by way of example with reference to the accompanying drawings, in which Figures 1 and 2 are schematic cross-sections of carriers according to the invention.

In Figure 1, a porous silica carrier 1 is 190 microns across and made up of silica microspheres 2 which are 8 microns in diameter. A porous coating of chitosan 3 encloses the silica particle providing an attractive surface for adhesion and growth of cells 4. The uneven surface is a desirable feature assisting adhesion and protecting the cells from surface shear stress in stirred reactors. Figure 2 additionally has crevices 5 as shown.

The Figure 1 microcarrier was used at a concentration of 2 mg/ml in the culture of mammalian cells F2002 which are 10 x 15 microns. After 8 days incubation at 37C a seven-fold increase in cell numbers was observed.

Further experiments on other carriers according to the invention were performed as follows.

The carriers were sterilised by autoclaving at 7.6 x $10^5$ Pa (110 psi) for 10 minutes in PBS (phosphate buffered saline).

Cultures were set up in 125 ml spinner flasks, with 50 ml of medium, cells at 0.5 x $10^5$/ml and an equal volume of carriers for each test. Flasks were stirred for 3-minute sessions once every 30 minutes at 50 rpm for 3 hours, then overnight at 42 rpm.

After 24 hours 10 ml of medium and carrier was removed, settled and supernatant medium removed. The carier was resuspended in PBS, pelleted and suspended in 2 ml trypsin versene for 15 minutes with occasional agitation. The supernatant was retained and the carrier was washed and resuspended in PBS. After settling, the supernatant was removed and added to the previous. Cells were spun down, suspended in 0.5 ml PBS and counted.

Results were expressed in terms of an Adhesion Coefficient equal to the number of cells recovered from the carrier divided by the number of cells seeded.

Adhesion coefficients greater than 1 are evidence of successful cell division, indeed are evidence that the cells adhered to the carriers with a high proportion of success or that division was so rapid even in 24 hours that it made up for any initial adhesion losses. For this reason, differences of up to about 20% in this Coefficient are not significant.

In the following experiments, the following carriers were used, and are identified by their serial numbers.

| Carrier serial number | Polymer coating | Average carrier diameter (microns) | Average microsphere diameter (microns) | Volume porosity (ml/ml) | Weight porosity (ml/g) | Density (g/cm³) | Wet Density (g/cm³) | Minimum stirring speed* (rpm) |
|---|---|---|---|---|---|---|---|---|
| ST 250 S | Polystyrene | 190 | 3 | 0.82 | 2.68 | 0.32 | 1.13 | 42 |
| ST 250 A | Cellulose acetate | 190 | 3 | 0.82 | 2.68 | 0.32 | 1.13 | 42 |
| ST 250 H | Chitosan | 190 | 3 | 0.82 | 2.68 | 0.32 | 1.13 | 42 |
| ST 400 H | Chitosan | 325 | 3 | 0.82 | 2.68 | 0.32 | 1.13 | 48 |
| ST 130 H | Chitosan | 90 | 3 | 0.82 | 2.68 | 0.32 | 1.13 | 38 |
| AL 250 S | Chitosan | 190 | 8 | 0.83 | 2.45 | 0.34 | 1.17 | 42 |
| AL 250 L | Chitosan | 190 | 15 | 0.81 | 2.41 | 0.34 | 1.15 | 42 |
| ST 250 A8 | Cellulose acetate | 190 | 3 | 0.78 | 2.07 | 0.41 | 1.25 | 50 |
| ST 250 A9 | Cellulose acetate | 190 | 3 | 0.51 | 0.56 | 0.91 | 1.43 | 62 |

*In later experiments, 50 rpm was used. The speed of 62 rpm used with ST 250 A9 is high but not exceptionally so.

When we speak of microspheres of 3 microns, we mean that all are within the range 2-4 microns. As for microspheres of 8 microns, all are within the range 3-30 microns with of course a peak at 8 microns, and microspheres of 15 microns are all within the range 7-40 microns.

Experiment 1

4

Effect of the nature of the surface coating

Three types of silica core, all with the same particle size, microsphere diameter (hence surface texture), porosity and density were coated with (a) polystyrene - ST 250 S, (b) cellulose acetate - ST 250 A and (c) chitosan - ST 250 H. These were tested for cell adhesion.

| Carrier serial number | Coating | Surface charge of coating in microequivalents per gram | Adhesion coefficient |
|---|---|---|---|
| ST 250 S | Polystyrene | 0.22 | 0.73 |
| ST 250 A | Cellulose acetate | 0.24 | 0.98 |
| ST 250 H | Chitosan | 0.85 | 2.03 |

The surface charge in the same units of uncoated silica is 0.24 (0.20 if it has been treated at 900C). Chitosan has well over $10^{15}$ charges per square centimetre and polystyrene some $2\text{-}10 \times 10^{14}$ charges per square centimetre.

The superiority of chitosan is apparent.

Experiment 2

Effect of the surface area to volume ratio

Three types of carriers, all with the same surface texture, porosity, density and coating but with different average particle size were tested for cell adhesion. The coating was chitosan in each case, this having been seen to be advantageous from Experiment 1. The microbead size was 3 microns in each case.

| Carrier serial number | Carrier size (micrometres) | Surface area/volume ratio (micron$^{-1}$) | Total surface area available (m$^2$) | Adhesion Coefficient |
|---|---|---|---|---|
| ST 400 H | 250-400 | 0.036 | 3.2 | 1.00 |
| ST 250 H | 130-250 | 0.064 | 5.4 | 2.03 |
| ST 130 H | 50-130 | 0.133 | 11.6 | 1.20 |

While the Adhesion Coefficient would be expected to relate to the total surface area available in the culture, the radius of curvature of the smallest carriers may impede adhesion. There may also be overall reasons connected with the dynamics of the reactor for preferring the 130-250 micron size range.

Experiment 3

Effect of surface texture

Three types of carrier were made all with the same overall size, porosity, density and coating but made up of aggregates of different sized microspheres, and were tested for cell adhesion. The cells were oblate 10 x 15 microns.

| Carrier serial number | Average microsphere size (microns) | Adhesion coefficient |
|---|---|---|
| ST 250 H | 3 | 2.03 |
| AL 250 S | 8 | 3.10 |
| AL 250 L | 15 | 2.80 |

There appears to be a preferred surface textural dimension for rapid cell adhesion.

Experiment 4

Effect of carrier density

5

Carriers of different density were made by heat treatment of the usual silica core at 800C and 900C. Samples tested for cell adhesion had the same particle size, surface texture and coating (cellulose acetate) but it was not possible to retain the same porosity; fusion starts to occur but the individual microbeads retain their identity up to about 1050C. However, the difference in porosity was not expected to influence cell adhesion. Compared with 800C, the 900C treatment removes hydroxyl groups and increases the acidity of the silica.

| Carrier Serial number | Treatment temperature °C | Dry density | Wet density | Adhesion coefficient |
|---|---|---|---|---|
| ST 250 A | - | 0.32 | 1.13 | 0.48 |
| ST 250 A8 | 800 | 0.41 | 1.25 | 0.58 |
| ST 250 A9 | 900 | 0.91 | 1.43 | 3.40 |

Visual inspection of the last sample showed that the growing cells 'looked' healthy. The effect may be due to density or surface acidity or both.

**Claims**

1. A carrier for living cells, comprising an indented body bearing an organic polymer coating providing an attractive surface for cell adhesion, characterised in that the body is an aggregation of microparticles from 2 to 30 microns across, said indentations being formed by interstices between the microparticles.

2. A carrier according to Claim 1 wherein the body is generally rounded.

3. A carrier according to Claim 1 or 2, wherein the microparticles are from 5 to 15 microns across.

4. A carrier according to any preceding claim, wherein the organic polymer is borne on the exterior surface of the aggregation.

5. A carrier according to Claim 1, 2 or 3, wherein the microparticles are coated with the organic polymer and aggregated.

6. A carrier according to any preceding claim, wherein the body is from 30 to 110 microparticles across.

7. A carrier according to Claim 6, wherein the body is from 40 to 80 microparticles across.

8. A carrier according to any preceding claim, wherein the body is from 50 to 1000 microns across.

9. A carrier according to Claim 8, wherein the body is from 100 to 400 microns across.

10. A carrier according to Claim 9, wherein the body is from 125 to 250 microns across.

11. A carrier according to any preceding claim, wherein the body has a creviced surface, the crevices themselves also being indented.

12. A carrier according to Claim 11, wherein the crevices are from 3 to 10 indentations deep.

13. A carrier according to Claim 11 or 12, wherein the crevices are from 2 to 5 indentations wide.

14. A carrier according to any preceding claim, wherein the indentations are generally 1 to 20 microns across.

15. A carrier according to Claim 14, wherein the indentations are generally 2 to 10 microns across.

16. A carrier according to any preceding claim, wherein the organic polymer has a surface charge of at least $10^{15}$ charges/cm$^2$.

**17.** A carrier according to any preceding claim, wherein the organic polymer has an exchange capacity of at least 0.2 microequivalents/gram.

**18.** A carrier according to Claim 17, wherein the organic polymer has an exchange capacity of at least 0.5 microequivalents/gram.

**19.** A carrier according to any preceding claim, wherein the organic polymer layer is porous.

**20.** A carrier according to any of Claims 1 to 18, wherein the organic polymer layer is non-porous.

**21.** A carrier according to any preceding claim, wherein the organic polymer layer is from 1 to 30 molecules thick.

**22.** A carrier according to any preceding claim, wherein the organic polymer layer comprises amino or hydroxyl groups.

**23.** A carrier according to any preceding claim, wherein the organic polymer is agar, agarose, alginic acid, casein, cellulose or its acetate or nitrate, chitosan, collagen, dextran, gelatin, glycogen, kappa-carrageenan, pectin, polystyrene, polyvinyl acetate, guar gum, gum tragacanth or gum xanthan or derivatives of these.

**24.** A carrier according to Claim 23, wherein the organic polymer is cross-linked with formaldehyde or glutaraldehyde.

**25.** A carrier according to Claim 23 or 24, wherein the organic polymer is chitosan.

**26.** A carrier according to any preceding claim, wherein the body is of an inorganic material.

**27.** A carrier according to Claim 26, wherein the body is of silica or aluminosilicate, which may be surface treated, heated or methylated, or of a carbide or a metal oxide.

**28.** A carrier according to any of Claims 1 to 25, wherein the body is of gelatin.

**29.** A carrier according to any preceding claim, wherein the interstices account for 30-80% of the volume of the body.

**30.** A method of culturing cells comprising providing the cells with nutrient and carriers according to any preceding claim.

**31.** A method according to Claim 30, wherein the microparticles are from half to twice the diameter of the cells.

**32.** A method according to Claim 30 or 31, further comprising harvesting the cells by degrading the organic polymer.

**33.** A method according to Claim 30, 31 or 32, wherein the density of the carriers in use is from 1.1 to 1.6 $g/cm^3$.

**34.** A method according to Claim 33, wherein the density of the carriers in use is from 1.2 to 1.5 $g/cm^3$.

**35.** A method according to any of Claims 30 to 34, wherein the organic polymer is insoluble in nutrient medium.

**36.** A method according to any of Claims 30 to 35, wherein the cells are animal, plant or fungal cells.

**Revendications**

**1.** Porteur pour des cellules vivantes, comprenant un corps dentelé revêtu d'un polymère organique

7

EP 0 245 338 B1

procurant une surface attirante pour l'adhérence des cellules, caractérisé en ce que le corps est un agrégat de microparticules ayant 2 à 30 $\mu$ m en travers, ses dentelures étant formées par des interstices entre les microparticules.

**2.** Porteur selon la revendication 1, dans lequel le corps est généralement arrondi.

**3.** Porteur selon la revendication 1 ou la revendication 2, dans lequel les microparticules ont de 5 à 15 $\mu$ m en travers.

**4.** Porteur selon l'une des revendications précédentes, dans lequel le polymère organique est porté sur la surface extérieure de l'agrégat.

**5.** Porteur selon l'une des revendications 1 à 3, dans lequel les microparticules sont revêtues par le polymère organique et agrégées.

**6.** Porteur selon l'une des revendications précédentes, dans lequel le corps a entre 30 et 110 microparticules en travers.

**7.** Porteur selon la revendication 6, dans lequel le corps a entre 40 et 80 microparticules en travers.

**8.** Porteur selon l'une des revendications précédentes, dans lequel le corps a 50 à 1000 $\mu$ m en travers.

**9.** Porteur selon la revendication 8, dans lequel le corps a 100 à 400 $\mu$ m en travers.

**10.** Porteur selon la revendication 9, dans lequel le corps a 125 à 250 $\mu$ m en travers.

**11.** Porteur selon l'une des revendications précédentes, dans lequel le corps a une surface crevassée, les crevasses elles-mêmes étant également dentelées.

**12.** Porteur selon la revendication 11, dans lequel les crevasses ont de 3 à 10 dentelures de profondeur.

**13.** Porteur selon la revendication 11 ou la revendication 12, dans lequel les crevasses ont de 2 à 5 dentelures de largeur.

**14.** Porteur selon l'une des revendications précédentes, dans lequel les dentelures ont en général 1 à 20 $\mu$ m en travers.

**15.** Porteur selon la revendication 14, dans lequel les dentelures ont généralement 2 à 10 $\mu$ m en travers.

**16.** Porteur selon l'une des revendications précédentes, dans lequel le polymère organique a une charge de surface d'au moins $10^{15}$ charges/cm2.

**17.** Porteur selon l'une des revendications précédentes, dans lequel le polymère organique a une capacité d'échange d'au moins 0,2 micro-équivalent /gramme.

**18.** Support selon la revendication 17, dans lequel le polymère organique a une capacité d'échange d'au moins 0,5 micro-équivalent/gramme.

**19.** Porteur selon l'une des revendications précédentes, dans lequel la couche de polymère organique est poreuse.

**20.** Porteur selon l'une des revendications 1 à 18, dans lequel la couche de polymère organique est non poreuse.

**21.** Porteur selon l'une des revendications précédentes, dans lequel la couche de polymère organique a 1 à 30 molécules d'épaisseur.

**22.** Porteur selon l'une des revendications précédentes, dans lequel la couche de polymère organique

8

comprend des groupes aminés ou hydroxyles.

23. Porteur selon l'une des revendications précédentes, dans lequel le polymère organique est l'un des suivants : agar, agarose, acide algénique, caséine, cellulose ou son acétate ou nitrate, chitosane, collagène, dextrane, gélatine, glycogène, kappa-carragénane, pectine, polystyrène, acétate de polyvinyle, gomme de guar, gomme adragante ou gomme de xanthane, ou leurs dérivés.

24. Porteur selon la revendication 23, dans lequel le polymère organique est réticulé avec du formaldéhyde ou du glutaraldéhyde.

25. Porteur selon la revendication 23 ou la revendication 24, dans lequel le polymère organique est le chitosane.

26. Porteur selon l'une des revendications précédentes, dans lequel le corps est en un matériau inorganique.

27. Porteur selon la revendication 26, dans lequel le corps est en silice ou alumino silicate, lequel peut être traité en surface, chauffé ou méthylé, ou le corps est en carbure ou oxyde métallique.

28. Porteur selon l'une des revendications 1 à 25, dans lequel le corps est en gélatine.

29. Porteur selon l'une des revendications précédentes, dans lequel les interstices comptent pour 30 à 80% du volume du corps.

30. Méthode pour cultiver des cellules, consistant à procurer aux cellules des substances nutritives et des porteurs selon l'une des revendications précédentes.

31. Méthode selon la revendication 30, dans laquelle les microparticules ont un diamètre compris entre la moitié et le double du diamètre des cellules.

32. Méthode selon la revendication 30 ou la revendication 31, consistant en outre à récolter les cellules en dégradant le polymère organique.

33. Méthode selon l'une des revendications 30 à 32, dans laquelle la densité des porteurs en service est comprise entre 1,1 et 1,6 g/cm3.

34. Méthode selon la revendication 33, dans laquelle la densité des porteurs en service est comprise entre 1,2 et 1,5 g/cm3.

35. Méthode selon l'une des revendications 30 à 34, dans laquelle le polymère organique est insoluble dans le milieu nutritif.

36. Méthode selon l'une des revendications 30 à 35, dans laquelle les cellules sont des cellules animales, végétales ou fongiques.

**Patentansprüche**

1. Träger für lebende Zellen, umfassend einen gekerbten Körper mit einer Beschichtung aus einem organischen Polymeren, die eine attraktive Oberfläche für Zelladhäsion bietet, dadurch gekennzeichnet, daß der Körper eine Aggregation von Mikroteilchen mit einem Durchmesser von 2 bis 30 $\mu$m ist und die Kerben durch Zwischenräume zwischen den Mikroteilchen gebildet werden.

2. Träger nach Anspruch 1, worin der Körper allgemein abgerundet ist.

3. Träger nach Anspruch 1 oder 2, worin die Mikroteilchen einen Durchmesser von 5 bis 15 $\mu$m aufweisen.

4. Träger nach einem der vorangehenden Ansprüche, worin das organische Polymer auf der Außen-Oberfläche der Aggregation vorhanden ist.

5. Träger nach Anspruch 1, 2 oder 3, worin die Mikroteilchen mit dem organischen Polymer beschichtet und aggregiert sind.

6. Träger nach einem der vorangehenden Ansprüche, worin der Körper im Querschnitt 30 bis 110 Mikroteilchen aufweist.

7. Träger nach Anspruch 6, worin der Körper im Querschnitt 40 bis 80 Mikroteilchen aufweist.

8. Träger nach einem der vorangehenden Ansprüche, worin der Körper einen Durchmesser von 50 bis 1000 $\mu$m aufweist.

9. Träger nach Anspruch 8, worin der Körper einen Durchmesser von 100 bis 400 $\mu$m aufweist.

10. Träger nach Anspruch 9, worin der Körper einen Durchmesser von 125 bis 250 $\mu$m aufweist.

11. Träger nach einem der vorangehenden Ansprüche, worin der Körper eine gespaltene/gerissene Oberfläche aufweist und die Spalten/Risse ihrerseits gekerbt sind.

12. Träger nach Anspruch 11, worin die Spalten/Risse 3 bis 10 Kerben tief sind.

13. Träger nach Anspruch 11 oder 12, worin die Spalten/Risse 2 bis 5 Kerben breit sind.

14. Träger nach einem der vorangehenden Ansprüche, worin die Kerben allgemein einen Querschnitt von 1 bis 20 $\mu$m aufweisen.

15. Träger nach Anspruch 14, worin die Kerben allgemein einen Querschnitt von 2 bis 10 $\mu$m aufweistn.

16. Träger nach einem der vorangehenden Ansprüche, worin das organische Polymer eine Oberflächenbeladung von mindestens $10^{15}$ Beladungen/cm$^2$ aufweist.

17. Träger nach einem der vorangehenden Ansprüche, worin das organische Polymer eine Austauschkapazität von mindestens 0,2 $\mu$Äq/g aufweist.

18. Träger nach Anspruch 17, worin das organische Polymer eine Austauschkapazität von mindestens 0,5 $\mu$Äq/g aufweist.

19. Träger nach einem der vorangehenden Ansprüche, worin die Schicht aus organischem Polymer porös ist.

20. Träger nach einem der Ansprüche 1 bis 18, worin die Schicht aus organischem Polymer nicht porös ist.

21. Träger nach einem der vorangehenden Ansprüche, worin die Schicht aus organischem Polymer 1 bis 30 Moleküle stark ist.

22. Träger nach einem der vorangehenden Ansprüche, worin die Schicht aus organischem Polymer Amino- oder Hydroxylgruppen aufweist.

23. Träger nach einem der vorangehenden Ansprüche, worin das organische Polymer Agar, Agarose, Alginsäure, Casein, Cellulose oder sein Acetat oder Nitrat, Chitosan, Collagen, Dextran, Gelatine, Glykogen, kappa-Carrageenan, Pektin, Polystyrol, Polyvinylacetat, Guargummi, Tragantgummi oder ein Derivat dieser Stoffe ist.

24. Träger nach Anspruch 23, worin das organische Polymer mit Formaldehyd oder Glutaraldehyd vernetzt ist.

10

**25.** Träger nach Anspruch 23 oder 34, worin das organische Polymer Chitosan ist.

**26.** Träger nach einem der vorangehenden Ansprüche, worin der Körper aus einem anorganischen Material besteht.

**27.** Träger nach Anspruch 26, worin der Körper aus Kieselsäure oder Aluminiumsilicat, die oberflächenbehandelt, erhitzt oder methyliert sein können, oder aus einem Carbid oder einem Metalloxid besteht.

**28.** Träger nach einem der Ansprüche 1 bis 25, worin der Körper aus Gelatine besteht.

**29.** Träger nach einen der vorangehenden Ansprüche, worin die Zwischenräume 30-80 Vol-% des Körpers ausmachen.

**30.** Verfahren zum Züchten von Zellen, dadurch gekennzeichnet, daß den Zellen Nährstoffe und Träger nach einem der vorangehen den Ansprüche bereitgestellt werden.

**31.** Verfahren nach Anspruch 30, worin die Mikroteilchen 1/2- bis 2-mal den Durchmesser der Zellen ausmachen.

**32.** Verfahren nach Anspruch 30 oder 31, zusätzliche dadurch gekennzeichnet, daß man die Zellen durch Abbau des organischen Polymeren erntet.

**33.** Verfahren nach Anspruch 30, 31 oder 32, worin die Dichte der eingesetzten Träger 1,1 bis 1,6 g/cm$^3$ ausmacht.

**34.** Verfahren nach Anspruch 33, worin die Dichte der Träger 1,2 bis 1,5 g/cm$^3$ ausmacht.

**35.** Verfahren nach einem der Ansprüche 30 bis 34, worin das organische Polymer im Nährmedium unlöslich ist.

**36.** Verfahren nach einem der Ansprüche 30 bis 35, worin die Zellen Zellen von Tieren, Pflanzen oder Pilzen sind.

Fig. 1

Fig. 2